# EUROPÄISCHE PATENTSCHRIFT

(11) **EP 2 241 349 B1**
(45) Veröffentlichungstag und Bekanntmachung des Hinweises auf die Patenterteilung: **14.05.2014**
(21) Anmeldenummer: 10156089.4
(22) Anmeldetag: 10.03.2010
(51) Int. Cl.: A61N 1/375, H01R 13/52

(54) **Dichtelement für eine Steckverbindung mit verringertem Fügewiderstand**
Seal element for a connector with reduced joint resistance
Elément d'étanchéité pour une fiche de raccordement doté d'une moindre résistance d'assemblage

(30) Priorität: 15.04.2009 DE 102009002398
(43) Veröffentlichungstag der Anmeldung: 20.10.2010
(73) Patentinhaber: Biotronik CRM Patent AG, 6341 Baar (CH)
(72) Erfinder: Flach, Erhard, 12305, Berlin (DE); Rebentisch, Ronald, 10967, Berlin (DE)
(74) Vertreter: Lindner-Vogt, Karin L.

(56) Entgegenhaltungen:
- EP-A1- 0 052 879
- US-A- 5 324 312
- US-A1- 2007 213 781
- US-A1- 2009 012 576

## Beschreibung

Die vorliegende Erfindung betrifft ein Dichtelement sowie eine Steckverbindung mit einem Dichtelement zur Verwendung an oder in einem implantierten medizinischen Gerät (IMD), das einen verringerten mechanischen Fügewiderstand beim Fügen von Stecker und Steckerbuchse aufweist.

Bekannte Dichtelemente und Steckverbindungen haben den Nachteil, dass es beim Fügen von Stecker und Steckerbuchse zu Kraftspitzen kommt, und zwar besonders dann, wenn die Haftreibung beim Auftreffen des Dichtelementes oder der Dichtelemente auf die ihnen zugeordneten Dichtflächen überwunden und die häufig auftretende notwendige Verformungsenergie zum Passieren der Eintrittsöffnungen der Dichtflächen aufgebracht werden muss. Diese Kraftspitzen können bei IMDs zu einer Beschädigung des Steckers, der Steckerbuchse und/oder von Leitungen, die am Stecker angebracht sind, führen (zum Beispiel durch Abknicken der Leitung).
Im Weiteren wird mit dem Begriff "Haftreibung" die Haftreibung und/oder die Kraft, die zur Verformung des Dichtelementes notwendig ist, verstanden. Auch ist das Dichtelement nicht auf ein einfaches Dichtelement beschränkt, sondern kann sich auch auf den dichtenden Teil einer größeren Struktur beziehen.

Bisherige Lösungsansätze optimieren vor allem die Dichtfähigkeit der Dichtelemente, minimieren dabei häufig die Dichtfläche und gehen auf das Fügen nur insoweit ein, dass die Außenkanten und oder Innenkanten der Dichtelemente abgerundet werden und oder die Reibung reduzierende Additive und/oder Beschichtungen verwendet werden.

Die EP0052879A1 offenbart eine einfache Dichtungsanordung für Implantate.

Die US5324312A zeigt eine Verbindungsvorrichtung für Implantate.

Die US2009/012576A1 beschreibt einen Header für Implantate mit Dichtelementen.

Die US2007/213781A1 offenbart unter anderem Schmiermittel zur Verwendung bei Steckverbindungen von Implantaten.

Durch die vorliegende Erfindung sollen ein in Bezug auf die Vermeidung von Kraftspitzen beim Fügen optimiertes Dichtelement und eine optimierte Steckverbindung mit dem Dichtelement bereitgestellt werden.

Gemäß der vorliegenden Erfindung wird ein Dichtelement zum Abdichten von Steckverbindungen eines implantierbaren medizinischen Geräts (IMD) bereitgestellt, bei dem das Dichtelement in einer Ebene eine erste Ausdehnung und eine zweite Ausdehnung senkrecht zur Ebene aufweist, eine Öffnung besitzt, die in der Ebene liegt und sich über die zweite Ausdehnung erstreckt und bei dem das Dichtelement so hergestellt ist, dass es ohne Krafteinwirkung geschert ist, so dass ein proximales Ende des Dichtelementes (21b, 61d) oder distales Ende des Dichtelementes (21a, 61c) beim Einfügen des proximalen Endes eines Steckers (20a, 60) in eine Steckerbuchse (11, 65) eine Dichtfläche des Steckers (14, 60c) zuerst berührt und erst mit weiterem Einfügen der Rest des Dichtelementes mit der Dichtfläche (14, 60c) in Berührung kommt und zuletzt ein distales Ende des Dichtelementes (21a, 61c) oder proximales Ende des Dichtelements (21b, 61d) das proximale Ende der Dichtfläche (14, 60c) passiert.
Die Formulierung geschert bezieht sich sowohl auf ein oder mehrere Sicherungen und/oder Streckungen und/oder Versetzungen der Grundform. Das Dichtelement ist so hergestellt, dass es ohne Krafteinwirkung die gescherte Form hat. Der Begriff Steckverbindung umfasst sowohl elektrische Steckverbindungen als auch Steckverbindungen für hohle Leitungen, wie zum Beispiel für Flüssigkeiten.

Des Weiteren kann das Dichtelement derart geschert sein, dass das gescherte Dichtelement auf einem Teilstück parallel zur zweiten Ausdehnung verläuft. In einer bevorzugten Ausführungsform kann das Dichtelement die Form eines parallel zu einer zweiten Ausdehnung gescherten Dichtringes aufweisen.

In einer weiteren bevorzugten Ausführung besteht das Dichtelement aus einem elastischen Material mit einer Shorehärte zwischen 15 ShA und 80 ShA bestimmt nach der DIN 53505 und DI N 7868.

In einer besonders bevorzugten Ausführung besteht das Dichtelement (21) aus einem Elastomer und/oder Polymer, vorzugsweise aus Silikonkautschuck oder Latex, Polyurethan oder Polyurethan-Copolymer mit Silikonanteil.
In einer weiteren bevorzugten Ausführung ist das Dichtelement mit einer hydrophoben und/oder hydrophilen Beschichtung zur Reduzierung der Gleitreibung versehen.

Ebenso wird gemäß der vorliegenden Erfindung eine Steckverbindung für ein IMD bereitgestellt, umfassend einen Stecker und eine Steckerbuchse, mit einem gescherten Dichtelement, dass das von der Steckerbuchse und dem proximalen Ende des Steckers gebildete Lumen von der Umgebung abdichtet. Das Dichtelement kann nach einer der oben beschriebenen Ausführungsformen ausgeführt sein.

In einer bevorzugten Ausführungsform kann das Dichtelement auf dem Stecker oder in der Steckerbuchse in einer Vertiefung sitzen, die geeignet, ist das Dichtelement teilweise aufzunehmen und auf dem Stecker oder in der Steckerbuchse zu fixieren. Alternativ kann das Dichtelement auch Teil eines größeren Elementes sein, und das größere Element fixiert das Dichtelement auf dem Stecker oder in der Steckerbuchse. Auch kann die Steckverbindung so ausgeführt sein, dass das gescherte Dichtelement auf einem Teilstück parallel zur Längsachse des Steckers verläuft. In einer besonders bevorzugten Ausführungsform weist der Stecker zylindrische Form auf und das gescherte Dichtelement hat die Form eines gescherten Dichtringes. In einer weiteren besonders bevorzugten Ausführungsform weist die Steckverbindung zwei oder mehr längs zur Achse der Steckverbindung gescherte Dichtelemente auf, welche das von der Steckerbuchse und dem proximalen Ende des Steckers gebildete Lumen und das oder die Lumen zwischen den Dichtelementen von der Umgebung abdichten. Bevorzugt wird auch, dass die Steckverbindung zwei oder mehr Dichtelemente aufweist, wobei die Lage der Dichtelemente auf dem Stecker oder in der Steckerbuchse so beschaffen ist, dass die Dichtelemente beim Einfügen des Steckers in die Steckerbuchse zeitlich versetzt mit einer ihnen zugeordneten Dichtfläche der Steckerbuchse oder des Steckers in Berührung kommen. Besonders bevorzugt wird, dass der Versatz so ausgeprägt ist, dass ein Dichtelement das distale Ende der ihm zugeordneten Dichtfläche beim Einfügen erst berührt, wenn das zuvor eingeführte Dichtelement vollständig das distale Ende der ihm zugeordneten Dichtfläche passiert hat, beziehungsweise der zeitliche Versatz so ausgeprägt ist, dass eine Dichtfläche eines Steckers das distale Ende eines ihr zugeordneten Dichtelements erst berührt, wenn die zuvor eingeführte Dichtfläche vollständig das proximale Ende des ihr zugeordneten Dichtelements passiert hat.

Ausführungsformen der vorliegenden Erfindung werden in exemplarischer Weise mit Bezug auf die angehängten Zeichnungen beschrieben, die zeigen:
- Fig. 1a: eine Seitenansicht eines Steckers mit zwei Dichtelementen aus dem bekannten Stand der Technik,
- Fig. 1b: die zu überwindenden Kräfte beim Fügen eines Steckers aus dem Stand der Technik in Bezug auf den beim Fügen zurückgelegten Weg,
- Fig. 2a: eine erfindungsgemäße Ausführung einer Steckverbindung mit einem gescherten Dichtelement,
- Fig. 2b: einen erfindungsgemäß gescherten Dichtring,
- Fig. 2c: die zu überwindenden Kräfte beim Fügen eines Steckers in eine Steckerbuche mit einem erfindungsgemäßen Dichtelement wie in Fig. 2a gezeigt in Bezug auf den beim Fügen zurückgelegten Weg,
- Fig. 3a: eine erfindungsgemäße Ausführung einer Steckverbindung mit zwei gescherten Dichtelementen,
- Fig. 3b: die zu überwindenden Kräfte beim Fügen eines Steckers mit erfindungsgemäßen Dichtelementen aus Fig. 3a in Bezug auf den beim Fügen zurückgelegten Weg,
- Fig. 4a: eine erfindungsgemäße Ausführung einer Steckverbindung mit einem in zwei Bereichen gescherten und in einem Bereich gestrecktes Dichtelement,
- Fig. 4b: die zu überwindenden Kräfte beim Fügen eines Steckers mit erfindungsgemäßem Dichtelement aus Fig. 4a in Bezug auf den beim Fügen zurückgelegten Weg,
- Fig. 5a: eine erfindungsgemäße Ausführung einer Steckverbindung mit zwei gescherten Dichtelementen mit unterschiedlichem Durchmesser auf einem Stecker mit zwei unterschiedlichen Durchmessern.
- Fig. 5b: die zu überwindenden Kräfte beim Fügen eines Steckers mit erfindungsgemäßen Dichtelementen aus Fig. 5a in Bezug auf den beim Fügen zurückgelegten Weg,
- Fig. 6: eine erfindungsgemäße Ausführung einer Steckverbindung mit zwei gescherten Dichtelementen mit unterschiedlichem Durchmesser in einer Steckerbuchse mit zwei unterschiedlichen Durchmessern.

Es wird ein Dichtelement und eine Steckverbindung mit mindestens einem Dichtelement bereitgestellt. Zum Zweck der Darstellung werden die Dichtelemente als Dichtring und die Steckverbindung als zylindrischer Stecker mit zylindrischer Steckerbuchse dargestellt, sie können aber auch in beliebiger anderer Form, wie, aber nicht beschränkt auf, Dichtscheiben oder mehreckige Dichtscheiben oder mehreckige Dichtringe oder mehreckige Stecker und oder Steckerbuchsen bereitgestellt werden. Auch wird zum besseren Verständnis auf die Darstellung von mehr als zwei Dichtelementen verzichtet, wobei dem Fachmann klar ist, dass für zum Beispiel, aber nicht beschränkt auf, mehrpolare Steckverbindungen unter Umständen mehr Dichtelemente benötigt werden.

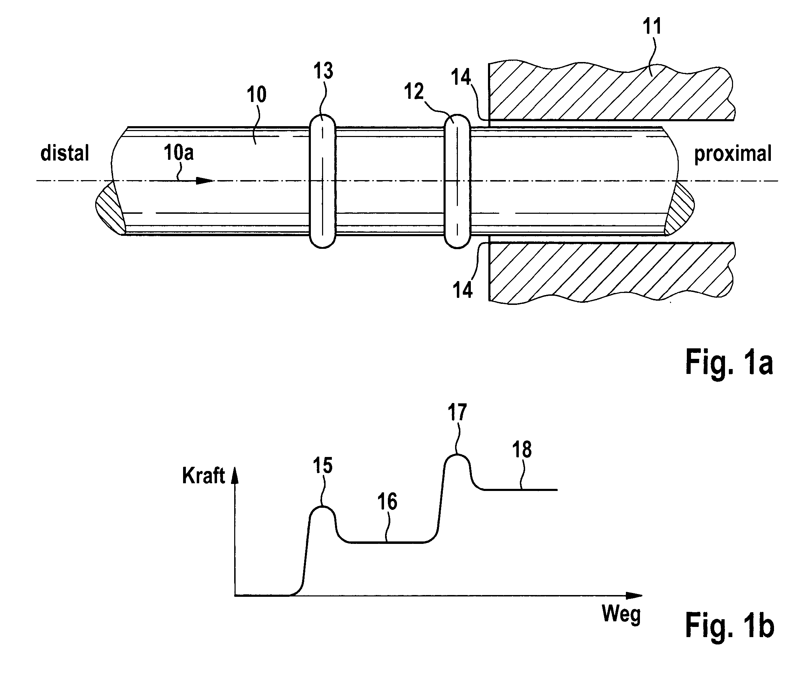

Bekannt aus dem Stand der Technik ist eine Steckverbindung wie in Fig. 1a abgebildet. Dabei sitzen auf einem Stecker 10 zwei Ringdichtungen 12 und 13. Das proximale Ende des Steckers wird in die Steckerbuchse 11 in Richtung des Pfeils 10a eingefügt. Die dabei zu überwindenden Kraftspitzen sind in Fig. 1b dargestellt. Die zu überwindenden Kräfte sind gegen den Weg des Steckers in die Steckerbuchse aufgetragen. Die auftretenden Kraftspitzen 15 und 17 entstehen durch die zu überwindenden Haftreibungen und Verformungskräfte, die entstehen wenn die Dichtelemente in die Dichtflächen 14 der Steckerbuchse 11 gepresst werden. Die Absenkungen 16 und 18 entstehen durch den Wegfall der zur Verformung aufzubringenden Kräfte und den Wegfall der Haftreibung nach dem vollständigen Eindringen des jeweiligen Dichtelements 12 oder 13 in die Dichtflächen 14.

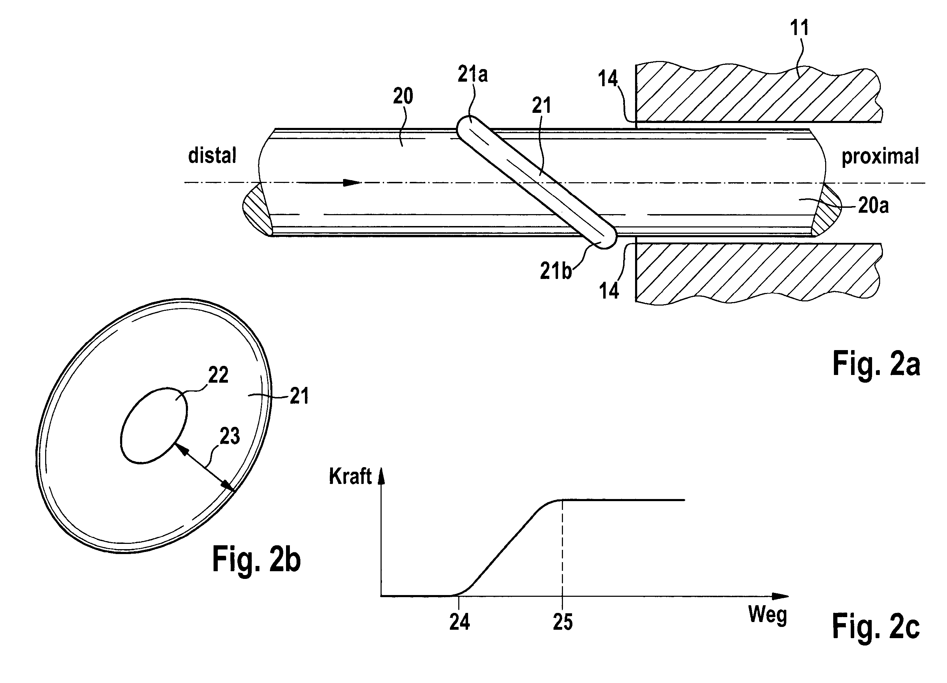

Eine erfindungsgemäße Steckverbindung mit einem erfindungsgemäßen Dichtelement ist in Fig. 2a dargestellt. Der Stecker 20 wird mit dem proximalen Ende 20a in die Steckerbuchse 11 eingefügt. Das Dichtelement 21 ist entlang der Steckerlängsachse geschert, so dass das proximale Ende des Dichtelementes 21 b beim Einfügen des proximalen Endes des Steckers 20a in die Steckerbuchse 11 die Dichtfläche 14 zuerst berührt und erst mit weiterem Einfügen der Rest des Dichtelementes mit der Dichtfläche 14 in Berührung kommt und zuletzt das distale Ende des Dichtelementes 21a das proximale Ende der Dichtfläche 14 passiert. In Fig. 2b ist ein Dichtelement 21 vor der Scherung abgebildet. Es weist eine Öffnung 22 auf, wobei die Fläche der Öffnung in der Fläche des Dichtelementes liegt und eine Achse senkrecht zur Fläche der Öffnung bildet und das Dichtelement eine konstante Dicke 23 an einer Position der Achse senkrecht zur Öffnung aufweist. In Fig. 2c ist die zu überwindende Kraft gegen den Weg des Steckers 20 in die Steckerbuchse 11 aufgetragen, wobei der Punkt 24 das Auftreffen des proximalen Abschnittes des Dichtelementes 21 b auf die Dichtfläche 14 und der Punkt 25 das vollständige Eintreten des distalen Endes des Dichtelementes 21a in die Dichtfläche 14 kennzeichnet. Dabei ist im Vergleich von Fig. 1b mit Fig. 2c das Fehlen der Kraftspitzen in Fig. 2c gut zu erkennen.

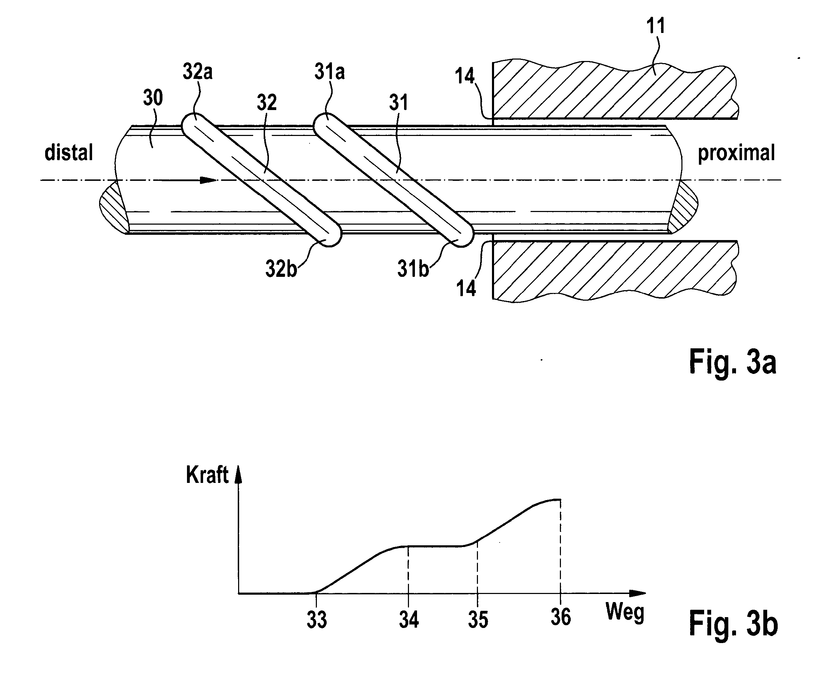

Fig. 3a zeigt einen Stecker 30 mit zwei erfindungsgemäßen, gescherten Dichtelementen 31 und 32 und die Steckerbuchse 11 mit dem distalen Ende der Dichtfläche 14. In Fig. 3b ist die zu überwindende Kraft gegen den Weg des Steckers 30 in die Steckerbuchse 11 aufgetragen, wobei der Punkt 33 das Auftreffen des proximalen Abschnittes des proximalen Dichtelementes 31 b auf die Dichtfläche 14, der Punkt 34 das vollständige Eintreten des distalen Endes des Dichtelementes 31a in die Dichtfläche 14, der Punkt 35 das Auftreffen des proximalen Abschnittes des distalen Dichtelementes 32b auf die Dichtfläche 14 und der Punkt 36 das vollständige Eintreten des distalen Endes des Dichtelementes 32a in die Dichtfläche 14 kennzeichnet.

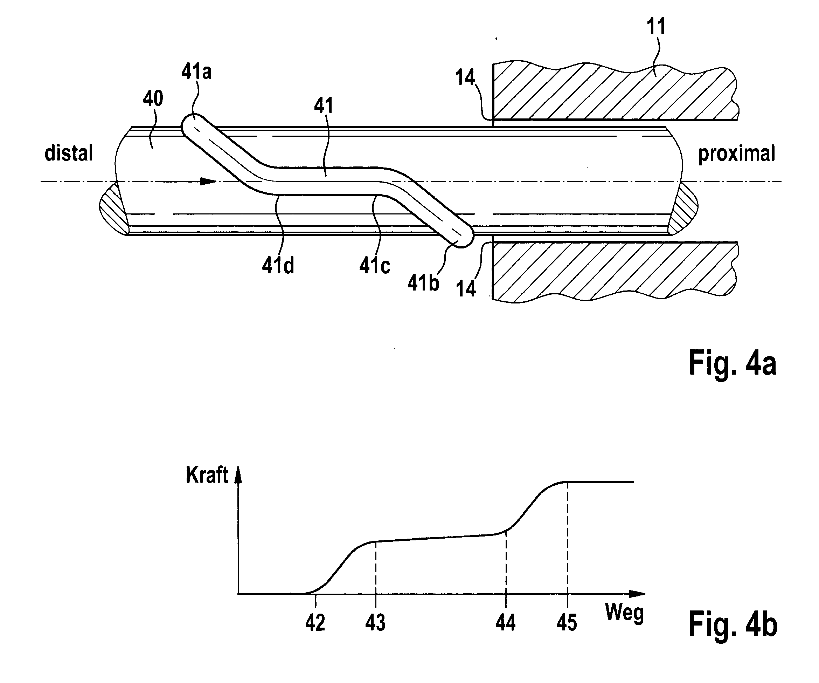

Fig. 4a zeigt ein erfindungsgemäß geschertes Dichtelement 41 auf einem Stecker 40. Das Dichtelement 41 ist in den zwei äußeren Bereichen geschert und im mittleren Bereich gestreckt, so dass das Dichtelement 41 auf einem Teilstück, zwischen 41c und 41d parallel zur Achse des Steckers 40 verläuft. Diese Anordnung führt zu der Gestalt des Graphen in Fig. 4b, in dem die zu überwindende Kraft gegen den Weg des Steckers 40 in die Steckerbuchse 11 aufgetragen ist. Der Punkt 42 im Graphen stellt den Wegabschnitt dar, in dem das proximale Ende des Dichtelementes 41 b auf die Dichtfläche 14 stößt, der Punkt 43 markiert den Übergang der Krümmung des Dichtelementes 41 c im Bereich, in dem das Dichtelement parallel zur Längsachse des Steckers verläuft. Der Punkt 44 markiert wiederum das Verlassen des parallel zur Längsachse des Steckers 40 verlaufenden Bereichs 41d des Dichtelementes 41 und der Punkt 45 das vollständige Eintreten des distalen Endes 41a des Dichtelementes 41 in die Dichtfläche 14.

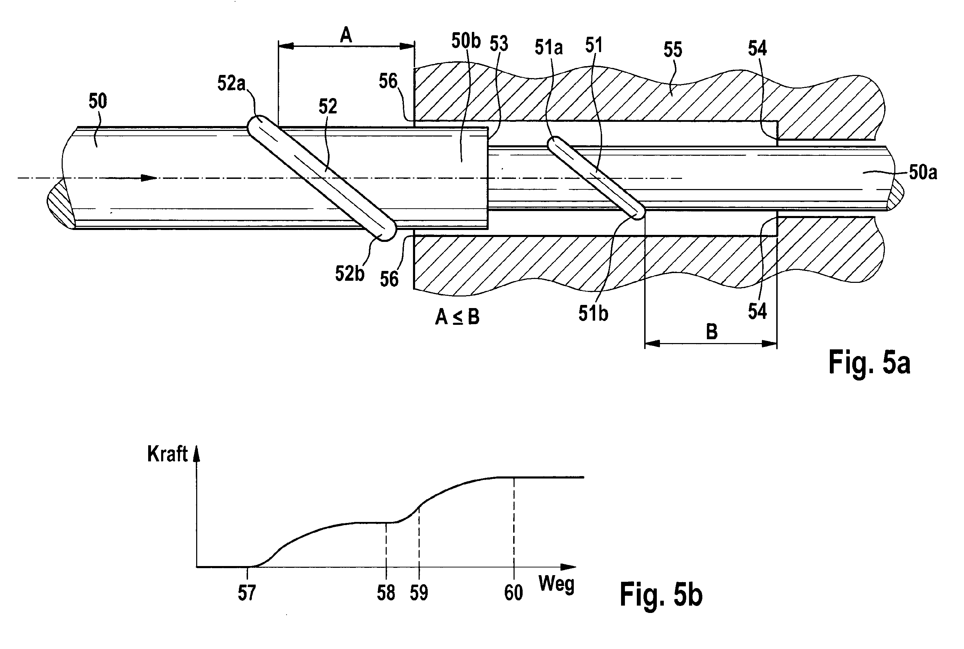

Fig. 5a zeigt eine besonders bevorzugte Form des Steckers 50 und der Steckerbuchse 55, bei der die zwei Dichtflächen 56 und 54 unterschiedliche Größen aufweisen, in die der jeweilige proximale Steckerabschnitt 50b und 50a passt. Die Dichtelemente 51 und 52 sind auf dem jeweiligen Steckerabschnitt 50a und 50b so angeordnet, dass sie beim Einfügen des Steckers 50 in die Steckerbuchse 55 zeitlich versetzt die jeweilige Dichtfläche 56 und 54 mit ihren proximalen Enden 52b und 51 b berühren, das heißt die Abstände A und B so gewählt sind, dass die Dichtelemente die jeweilig ihnen zugeordneten Dichtflächen zu unterschiedlichen Zeitpunkten berühren. Dabei ist der Abstand B des proximalen Endes 51 b des Dichtelementes 51 zum distalen Ende der Dichtfläche 54 im nicht eingefügten Zustand größer oder gleich dem Abstand des distalen Endes 52a des Dichtelementes 52 zum distalen Ende der Dichtfläche 56 ist.

In Fig. 5b ist die zu überwindende Kraft gegen den Weg des Steckers 50 in die Steckerbuchse 55 aufgetragen, wobei der Punkt 57 das Auftreffen des proximalen Abschnittes des proximalen Dichtelementes 51 b auf die Dichtfläche 54, der Punkt 58 das vollständige Eintreten des distalen Endes 51a des Dichtelementes 51,und der Punkt 59 das Auftreffen des proximalen Abschnittes des distalen Dichtelementes 52b auf die Dichtfläche 56 und der Punkt 60 das vollständige Eintreten des distalen Endes 52a des Dichtelementes 52 kennzeichnet.

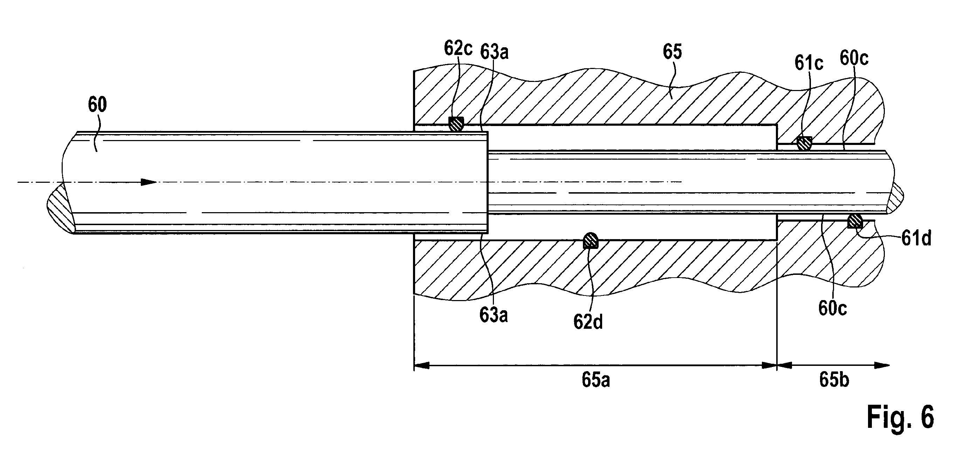

Fig. 6 zeigt eine besonders bevorzugte Form des Steckers 60 und der Steckerbuchse 65, bei der die zwei Dichtflächen 63a und 60c unterschiedliche Größen aufweisen, die in den jeweiligen Steckerbuchsenabschnitt 65a und 65b passen. Die Dichtelemente 61 und 62 sind in der Fig. 6 nur durch die Abschnitte 61c, 61d, 62c und 62d dargestellt, verlaufen in der Steckerbuchse 65' jedoch analog zu den Dichtelementen 51 und 52 auf dem Stecker in Fig. 5a. Die Dichtelemente 61 und 62 sind in dem jeweiligen Steckerbuchsenabschnitt 65a und 65b so angeordnet, dass die Dichtelemente beim Einfügen des Steckers 60 in die Steckerbuchse 65 zeitlich versetzt mit der jeweiligen Dichtfläche 63a und 60c in Berührung kommen.

## Patentansprüche

1. Dichtelement (21) zum Abdichten von Steckverbindungen eines implantierbaren medizinischen Geräts (IMD), wobei das Dichtelement (21)
- in einer Ebene eine erste Ausdehnung und eine zweite Ausdehnung senkrecht zur Ebene aufweist,
- eine Öffnung besitzt, die in der Ebene liegt und sich über die zweite Ausdehnung erstreckt,
**dadurch gekennzeichnet, dass** das Dichtelement so hergestellt ist, dass es ohne Krafteinwirkung geschert ist, so dass ein proximales Ende des Dichtelementes (21b, 61d) oder distales Ende des Dichtelementes (21a, 61c) beim Einfügen des proximalen Endes eines Steckers (20a, 60) in eine Steckerbuchse (11, 65) eine Dichtfläche des Steckers (14, 60c) zuerst berührt und erst mit weiterem Einfügen der Rest des Dichtelementes mit der Dichtfläche (14, 60c) in Berührung kommt und zuletzt ein distales Ende des Dichtelementes (21a, 61c) oder proximales Ende des Dichtelements (21b, 61d) das proximale Ende der Dichtfläche (14, 60c) passiert.

2. Dichtelement (41) nach Anspruch 1, **dadurch gekennzeichnet, dass** das gescherte Dichtelement (41) auf einem Teilstück parallel zur zweiten Ausdehnung verläuft.

3. Dichtelement (21) nach einem der vorhergehenden Ansprüche, **dadurch gekennzeichnet, dass** das Dichtelement (20) die Form eines parallel zu einer zweiten Ausdehnung gescherten Dichtringes aufweist.

4. Dichtelement (21) nach einem der vorhergehenden Ansprüche, **dadurch gekennzeichnet, dass** das Dichtelement aus einem elastischen Material mit einer Shorehärte zwischen 15 ShA und 80 ShA besteht.

5. Dichtelement (21) nach einem der vorhergehenden Ansprüche, **dadurch gekennzeichnet, dass** das Dichtelement (21) aus einem Elastomer und/oder Polymer, vorzugsweise aus Silikonkautschuck oder Latex, Polyurethan oder Polyurethan-Copolymer mit Silikonanteil, besteht.

6. Dichtelement (21) nach einem der vorhergehenden Ansprüche, **dadurch gekennzeichnet, dass** das Dichtelement (21) mit einer hydrophoben und/oder hydrophilen Beschichtung zur Reduzierung der Gleitreibung versehen ist.

7. Steckverbindung für ein IMD, umfassend einen Stecker und eine Steckerbuchse, **gekennzeichnet dadurch, dass** das gescherte Dichtelement (21) nach einem der vorhergehenden Ansprüche 1 bis 6, das von der Steckerbuchse (11) und dem proximalen Ende des Steckers (20a) gebildete Lumen von der Umgebung abdichtet.

8. Steckverbindung nach Anspruch 7, **dadurch gekennzeichnet, dass** das Dichtelement (21) auf dem Stecker (20) oder in der Steckerbuchse (65) in einer Vertiefung sitzt, die geeignet ist, das Dichtelement (21) teilweise aufzunehmen und auf dem Stecker (20) oder in der Steckerbuchse (65) zu fixieren.

9. Steckverbindung nach Anspruch 7, **dadurch gekennzeichnet, dass** das Dichtelement (21) Teil eines größeren Elementes auf dem Stecker (20) oder in der Steckerbuchse (65) ist und wobei das größere Element das Dichtelement auf dem Stecker (20) oder in der Steckerbuchse fixiert.

10. Steckverbindung nach einem der vorangegangenen Ansprüche, **dadurch gekennzeichnet, dass** das gescherte Dichtelement (41) auf einem Teilstück parallel zu einer Längsachse des Steckers verläuft.

11. Steckverbindung nach einem der vorangegangenen Ansprüche, **dadurch gekennzeichnet, dass** der Stecker (20) eine zylindrische Form und das gescherte Dichtelement (20) die Form eines gescherten Dichtringes aufweist.

12. Steckverbindung nach einem der vorangegangenen Ansprüche, **dadurch gekennzeichnet, dass** die Steckverbindung zwei oder mehr gescherte Dichtelemente (31, 32) aufweist.

13. Steckverbindung nach Anspruch 12, **dadurch gekennzeichnet, dass** die Steckverbindung zwei oder mehr Dichtelemente (51, 52) aufweist, wobei diese so angeordnet sind, dass sie beim Einfügen des Steckers (50) in die Steckerbuchse (55) zeitlich versetzt mit einer ihnen zugeordneten Dichtfläche (54, 56) der Steckerbuchse (55) oder des Steckers (65) in Berührung kommen.

14. Steckverbindung nach Anspruch 13, wobei der zeitliche Versatz so ausgeprägt ist, dass ein Dichtelement (51, 51 b) das distale Ende der ihm zugeordneten Dichtfläche (54) beim Einfügen erst berührt, wenn das zuvor eingeführte Dichtelement (52, 52a) vollständig das distale Ende der ihm zugeordneten Dichtfläche (56) passiert hat, beziehungsweise der zeitliche Versatz so ausgeprägt ist, dass eine Dichtfläche (63a) eines Steckers (60) das distale Ende eines ihr zugeordneten Dichtelements (62c) erst berührt, wenn die zuvor eingeführte Dichtfläche (60c) vollständig das proximale Ende des ihr zugeordneten Dichtelements (61 d) passiert hat.

## Claims

1. A sealing element (21) for sealing plug connections of an implantable medical device (IMD), wherein the sealing element (21)
- comprises a first expansion in one plane and a second expansion perpendicular to the plane, and
- has an opening which lies in the plane and extends over the second expansion, **characterised in that** the sealing element is produced such that it is sheared without an application of force, such that firstly a proximal end of the sealing element (21b, 61d) or a distal end of the sealing element (21a, 61c) touches a sealing face of the plug (14, 60c) when the proximal end of a plug (20a, 60) is inserted into a plug socket (11, 65), the rest of the sealing element only comes into contact with the sealing face (14, 60c) when the plug is inserted further, and lastly a distal end of the sealing element (21a, 61 c) or proximal end of the sealing element (21b, 61d) passes the proximal end of the sealing face (14, 60c).

2. The sealing element (21) according to Claim 1, **characterised in that** a portion of the sheared sealing element (41) runs parallel to the second expansion.

3. The sealing element (21) according to one of the preceding claims, **characterised in that** the sealing element (20) has the form of a ring seal which is sheared parallel to a second expansion.

4. The sealing element (21) according to one of the preceding claims, **characterised in that** the sealing element consists of a resilient material with a Shore hardness between 15 ShA and 80 ShA.

5. The sealing element (21) according to one of the preceding claims, **characterised in that** the sealing element (21) consists of an elastomer and/or polymer, preferably of silicone rubber or latex, polyurethane or polyurethane copolymer with silicone content.

6. The sealing element (21) according to one of the preceding claims, **characterised in that** the sealing element (21) is provided with a hydrophobic and/or hydrophilic coating to reduce the sliding friction.

7. A plug connection for an IMD, comprising a plug and a plug socket, **characterised in that** the sheared sealing element (21) according to one of preceding Claims 1 to 6 seals the lumen formed by the plug socket (11) and the proximal end of the plug (20a) with respect to the environment.

8. The plug connection according to Claim 7, **characterised in that** the sealing element (21) sits on the plug (20) or in the plug socket (65) in an indentation that is suitable for partly receiving the sealing element (21) and for fixing the sealing element on the plug (20) or in the plug socket (65).

9. The plug connection according to Claim 7, **characterised in that** the sealing element (21) is part of a larger element on the plug (20) or in the plug socket (65), wherein the larger element fixes the sealing element on the plug (20) or in the plug socket.

10. The plug connection according to one of the preceding claims, **characterised in that** a portion of the sheared sealing element (41) runs parallel to a longitudinal axis of the plug.

11. The plug connection according to one of the preceding claims, **characterised in that** the plug (20) has a cylindrical form and the sheared sealing element (20) has the form of a sheared ring seal.

12. The plug connection according to one of the preceding claims, **characterised in that** the plug connection comprises two or more sheared sealing elements (31, 32).

13. The plug connection according to Claim 12, **characterised in that** the plug connection comprises two or more sealing elements (51, 52), wherein these are arranged such that, when the plug (50) is inserted into the plug socket (55), the two or more sealing elements (51, 52) come into contact with their respective assigned sealing faces (54, 56) of the plug socket (55) or of the plug (65) with a time difference.

14. The plug connection according to Claim 13, wherein the time difference is configured in such a manner that a sealing element (51, 51b) touches the distal end of its respective assigned sealing face (54) during insertion only when the previously inserted sealing element (52, 52a) has completely passed the distal end of its respective assigned sealing face (56), or the time difference is configured in such a manner that a sealing face (63a) of a plug (60) touches the distal end of its respective assigned sealing element (62c) only when a previously inserted sealing face (60c) has completely passed the proximal end of its respective assigned sealing element (61d).

## Revendications

1. Élément d'étanchéité (21) pour l'étanchéité de connexions par enfichage d'un appareil médical implantable (IMD), dans lequel l'élément d'étanchéité (21)
- comporte une première extension dans un plan et une deuxième extension perpendiculaire au plan,
- possède une ouverture située dans un plan et s'étendant sur la deuxième extension,
**caractérisé en ce que** l'élément d'étanchéité est fabriqué de manière à être cisaillé sans force, de sorte qu'une extrémité proximale de l'élément d'étanchéité (21b, 61a) ou une extrémité distale de l'élément d'étanchéité (21a, 61c) entre d'abord en contact avec une surface d'étanchéité du connecteur (14, 60c) lors de l'insertion de l'extrémité proximale d'un connecteur (20a, 60) dans une fiche femelle (11, 65), et le reste de l'élément d'étanchéité n'entre en contact avec la surface d'étanchéité (14, 60c) que lors d'une insertion supplémentaire, puis une extrémité distale de l'élément d'étanchéité (21a, 61c) ou une extrémité proximale de l'élément d'étanchéité (21b, 6 1 d) dépasse l'extrémité proximale de la surface d'étanchéité (14, 60c).

2. Élément d'étanchéité (21) selon la revendication 1, **caractérisé en ce que** l'élément d'étanchéité (41) cisaillé s'étend sur une partie parallèlement à la deuxième extension.

3. Élément d'étanchéité (21) selon l'une des revendications précédentes, **caractérisé en ce que** l'élément d'étanchéité (20) se présente sous la forme d'un anneau d'étanchéité cisaillé parallèlement à une deuxième extension.

4. Élément d'étanchéité (21) selon l'une des revendications précédentes, **caractérisé en ce que** l'élément d'étanchéité est constitué d'un matériau élastique avec une dureté de Shore entre 15 ShA et 80 ShA.

5. Élément d'étanchéité (21) selon l'une des revendications précédentes, **caractérisé en ce que** l'élément d'étanchéité (21) est constitué d'un élastomère et/ou d'un polymère, de préférence de caoutchouc de silicone ou de latex, de polyuréthane ou d'un copolymère de polyuréthane avec une part de silicone.

6. Élément d'étanchéité (21) selon l'une des revendications précédentes, **caractérisé en ce que** l'élément d'étanchéité (21) est pourvu d'un revêtement hydrophobe et/ou hydrophile pour réduire la friction de glissement.

7. Connexion par enfichage pour un IMD, comprenant un connecteur et une fiche femelle, **caractérisée en ce que** l'élément d'étanchéité cisaillé (21) selon l'une des revendications 1 à 6 isole le lumen formé par la fiche femelle (11) et l'extrémité proximale du connecteur (20a) par rapport à l'environnement.

8. Connexion par enfichage selon la revendication 7, **caractérisé en ce que** l'élément d'étanchéité (21) est logé sur le connecteur (20) ou dans la fiche femelle (65), dans un creux adapté pour accueillir partiellement l'élément d'étanchéité (21) et le fixer sur le connecteur (20) ou dans la fiche femelle (65).

9. Connexion par enfichage selon la revendication 7, **caractérisée en ce que** l'élément d'étanchéité (21) fait partie d'un élément plus grand sur le connecteur (20) ou dans la fiche femelle (65), et dans laquelle l'élément plus grand fixe l'élément d'étanchéité sur le connecteur (20) ou dans la fiche femelle.

10. Connexion par enfichage selon l'une des revendications précédentes, **caractérisée en ce que** l'élément d'étanchéité cisaillé (41) s'étend sur une partie parallèlement à un axe longitudinal du connecteur.

11. Connexion par enfichage selon l'une des revendications précédentes, **caractérisée en ce que** le connecteur (20) présente une forme cylindrique et l'élément d'étanchéité cisaillé (20) présente la forme d'un anneau d'étanchéité cisaillé.

12. Connexion par enfichage selon l'une des revendications précédentes, **caractérisée en ce que** la connexion par enfichage comporte deux ou plusieurs éléments d'étanchéité cisaillés (31, 32).

13. Connexion par enfichage selon la revendication 12, **caractérisée en ce que** la connexion par enfichage comporte deux ou plusieurs éléments d'étanchéité (51, 52), dans laquelle ceux-ci sont agencés de manière à entrer en contact de façon décalée dans le temps avec l'une de leurs surfaces d'étanchéité (54, 56) correspondantes de la fiche femelle (55) ou du connecteur (65), lors de l'insertion du connecteur (50) dans la fiche femelle (55).

14. Connexion par enfichage selon la revendication 13, dans laquelle le décalage dans le temps est aménagé de telle sorte que lors de l'insertion, un élément d'étanchéité (51, 51b) n'entre en contact avec l'extrémité distale de la surface d'étanchéité (54) correspondante que lorsque l'élément d'étanchéité (52, 52a) inséré précédemment a entièrement dépassé l'extrémité distale de la surface d'étanchéité (56) correspondante, ou dans laquelle le décalage dans le temps est aménagé de telle sorte qu'une surface d'étanchéité (63a) d'un connecteur (60) n'entre en contact avec l'extrémité distale d'un élément d'étanchéité (62c) correspondant que lorsque la surface d'étanchéité (60c) insérée précédemment a entièrement dépassé l'extrémité proximale de l'élément d'étanchéité (61d) correspondant.
